Europäisches Patentamt

European Patent Office

Office européen des brevets

(11) Veröffentlichungsnummer: **0 187 881**
**A1**

## (12) EUROPÄISCHE PATENTANMELDUNG

(21) Anmeldenummer: 85100379.8

(51) Int. Cl.⁴: **A 61 F 2/34**

(22) Anmeldetag: 16.01.85

(43) Veröffentlichungstag der Anmeldung:
23.07.86 Patentblatt 86/30

(84) Benannte Vertragsstaaten:
AT BE CH DE FR GB IT LI NL SE

(71) Anmelder: Orthoplant Endoprothetik GmbH
Leerkämpe 12
D-2800 Bremen 1(DE)

(72) Erfinder: Gebauer, Heinrich K.D., Prof.-Dr.
Industriestrasse 39
D-8000 München 60(DE)

(72) Erfinder: Schelhas, Klaus-Dieter
Semperstrasse 3
D-2000 Hamburg 60(DE)

(74) Vertreter: Eisenführ & Speiser
Martinistrasse 24
D-2800 Bremen 1(DE)

(54) Hüftpfanne.

(57) Eine Hüftpfanne (1) für eine Hüftgelenk-Endoprothese zur zementfreien Verankerung im menschlichen Acetabulum-gewebe besteht aus einer Kunststoff-Schale (4), die eine halbkugelförmige Außenfläche und eine einen halbkugelförmigen Hohlraum (8) bildende Innenfläche besitzt. Die Innenfläche ist exzentrisch zur Außenfläche angeordnet, wodurch ein dünnwandiger und ein dickwandiger Pfannenbereich entsteht. Zur Ermöglichung einer ausreichenden Formanpassung an das sich während der Patienten-Bewegungen ständig verformenden Acetabulum-Gewebes besitzt die Pfanne im dünnwandigen Bereich einen Schlitz (14), der sich wenigstens bis zur Symmetrieachse (9) der Innenfläche hin erstreckt.

FIG.1

EP 0 187 881 A1

# Beschreibung

Die Erfindung betrifft eine Hüftpfanne für eine Hüftgelenk-Endoprothese zur zementfreien Verankerung im menschlichen Acetabulumgewebe, bestehend aus einer Schale aus Kunststoff, die eine halbkugelförmige Außenfläche und eine einen halbkugelförmigen Hohlraum bildende Innenfläche besitzt, die exzentrisch zur Außenfläche angeordnet ist und dadurch einen dünnwandigen und einen dickwandigen Pfannenbereich bildet, und wobei an dem Rand der Pfanne eine Ausnehmung vorhanden ist.

Nachstehend auch kurz jeweils als "Pfanne" bezeichnete prothetische Acetabulumpfannen zur Aufnahme und damit zur Lagerung und Abstützung natürlicher oder insbesondere künstlicher im wesentlichen kugelförmiger Femurköpfe von Hüftgelenk-Endoprothesen sind seit Jahren in unterschiedlichsten Ausgestaltungen bekannt. Obwohl sich die bekannten Pfannen hinsichtlich diverser an sie zu stellender Anforderungen - beispielsweise hinsichtlich ihrer Gewebeverträglichkeit, ihrer Lauf- und Verschleißeigenschaften etc. - im Verlaufe der Entwicklung an sich gut und in steigendem Maße besser bewährt haben, bestehen hinsichtlich ihrer Verankerung im Knochengewebe nach wie vor Probleme, und zwar insbesondere bei zementfreier Implantierung, aber auch bei einer Verbindung der Außenseite der Pfanne mit dem entsprechend ausgefrästen Acetabulumgewebe mit sog. Knochenzement. Man hat zwar inzwischen Wege und Möglichkeiten gefunden, um auch bei einer zementfreien oder zumindest weitgehend zementfreien Implantation weitgehend sicherzustellen, daß sich die Außenseite der Pfanne mit dem Knochengewebe verbindet, doch hat sich gezeigt, daß es gerade bei den Pfannen von Hüftgelenk-Endoprothesen dennoch häufig zu Lockerungen kommt, deren Ursache bisher oft nicht erklärlich war.

Weiterhin bereiten die bekannten Pfannen oft bezüglich des Nervus femuralis und des diesen begleitenden Sehnenstranges, der sich gleichsam in der Art eines Stranges aus dem Beckenbereich zu dem den Femur umgebenden Muskeln hin erstrecken, gewisse Schwierigkeiten, da die in der natürlichen Acetabulumpfanne hierfür gleichsam als Durchlaß vorgesehene Gewebeaussparung bei den bekannten halbkugelförmigen Pfannenprothesen nicht mehr vorhanden ist, so daß sich zum einen bereits bei der Operation hinsichtlich der Führung dieses Stranges Probleme ergeben können und zum anderen insbesondere auch nach der Implantation unter den gegebenen Belastungs- und Bewegungsverhältnissen.

Die vorerwähnten Eigenschaften gelten auch für eine aus der DE-PS 18 06 323 bekannte Pfanne, bei welcher zur Schaffung eines gegenüber vorher bekannten Pfannen größeren Bewegungsspielraums sowie zur Vermeidung einer Gefahr von Ausrenkungen der Pfannenrand durch einen schrägen Anschnitt eines Teilabschnittes der Schale an zwei im stumpfen Winkel zueinander stehenden Ebenen liegt. Durch diesen Anschnitt wird außerdem verhindert, daß bei der Bein-Bewegung Weichteile oder Nervenstränge eingequetscht oder gegen den Rand der Hüftpfanne - in dem Anschnittsbereich - gedrückt werden können, wie dies bei den zuvor bekannten, nichtangeschnittenen Hüftpfannen der Fall war. Im übrigen ist diese bekannte Hüftpfanne aufgrund ihrer Form relativ starr, so daß durch die ständigen Bein-Bewegungen erhebliche Spannungen in der Pfanne auftreten, welche zur Lockerung der Hüftpfanne führen können. Außerdem verhindert die hohe Formsteifigkeit dieser bekannten Hüftpfanne während des Gehens oder anderer Belastungen einerseits eine ausreichend spannungsarme Formanpassung an den Hohlraum des Acetabulums, in das die Pfanne eingesetzt ist, sowie andererseits zur günstigen Krafteinleitung die erwünschte beständige satte Formanpassung an den sich bewegenden Femurkopf. Da sich das Becken beim Gehen oder anderen Belastungen ständig verformt, und da sich die bekannte formsteife Hüftpfanne diesen Verformungen nicht ausreichend anpassen kann, kann es zu den unerwünschten und gefürchteten Lockerungserscheinungen kommen.

Aus der DE-AS 28 22 585 ist ferner eine konzentrische Hüftpfanne aus Kunststoff bekannt, die in das Acetabulum einzementiert wird. Zur Hemmung der Übertragungen von Ringspannungen innerhalb der Hüftpfanne sind bei dieser Hüftpfanne meridial verlaufende Schlitze vorgesehen, die am Rand beginnen und einen vorgegebenen Abstand vor dem Zentrum der Hüftpfanne enden. Die meridialen Schlitze sind dabei gleichmäßig am Umfang der Pfanne verteilt.

Durch die meridialen Schlitze werden bei der Hüftpfanne gemäß der DE-AS 28 22 585 zwar die Ringspannungen innerhalb der Hüfte wirksam beseitigt, wodurch eine gute Verformbarkeit der Hüftpfanne und damit auch eine spannungsarme Anpassung der Hüftpfanne an das Acetabulumgewebe erzielt wird, welches sich unter den Bewegungen und Belastungen des Patienten ständig verformt. Nachteilig bei dieser bekannten Pfanne ist es jedoch, daß durch die meridialen Schlitze die Formsteifigkeit der Pfanne gerade auch dort vollständig beseitigt wird – nämlich überall in dem aufgeschnittenen breiten Randbereich – wo vom Femurkopf die Stützkräfte über die Hüftpfanne auf das Becken eingeleitet werden. Die Einleitung der Stützkräfte und damit eine starke Druckbeanspruchung erfolgt nämlich bei einer implantierten Hüftpfanne im oberen Abschnitt des Randbereiches, wo die aus der DE-AS 28 22 585 bekannte Hüftpfanne geschlitzt ist. Nachteilig ist es dabei, daß dann die vollen Stützkräfte im wesentlichen über ein – durch zwei benachbarte Schlitze begrenztes – Segment, d. h. über eine relativ kleine Fläche und somit eine große Flächenpressung auf das Becken übertragen werden.

Aufgabe der Erfindung ist es demgegenüber, eine Hüftpfanne gemäß dem Oberbegriff des neuen Hauptanspruches derart weiterzubilden, daß die Hüftpfanne einerseits im Bereich hoher Druckbeanspruchung eine ausreichende Formstabilität besitzt und andererseits insgesamt ausreichend spannungsarm verformbar ist.

Diese Aufgabe wird bei der Hüftpfanne der eingangs genannten Art erfindungsgemäß dadurch gelöst, daß die Ausnehmung im dünnwandigen Bereich der Pfanne in der Art eines Schlitzes ausgebildet ist, der sich wenigstens bis zur Symmetrieachse der Innenfläche hin erstreckt, wobei unter dem Begriff "Symmetrieachse" vor- und nachstehend die Achse verstanden wird, zu welcher die Halbkugelfläche des Hohlraums und/oder der Außenseite rotationssymmetrisch angeordnet ist.

Die Vorteile der Erfindung liegen insbesondere darin, daß die erfindungsgemäße Hüftpfanne aufgrund ihrer Formgebung zwei an sich gegenläufige Forderungen ideal miteinander vereinigt: Dadurch, daß eine schlitzförmige Ausnehmung vorgesehen ist, die sich mindestens bis zur Symmetrieachse des Innenhohlraums hin erstreckt, wird eine gute, spannungsarme Verformbarkeit der gesamten Hüftpfanne erzielt, so daß sich die Hüftpfanne an die Verformungen des Acetabulums spannungsarm anpassen kann. Erfindungsgemäß ist nun dieser lange Schlitz im dünnen Wandbereich der exzentrischen Hüftpfanne angeordnet, während der dicke Wandbereich der Pfanne keine Ausnehmungen besitzt und daher lokal eine hohe Formstabilität aufweist. Da die Stützkräfte - bei stehendem oder gehendem Patienten - hauptsächlich im dickwandigen Randbereich der Hüftpfanne eingeleitet werden, und da dieser Bereich - erfindungsgemäß - keine Ausnehmungen enthält, werden diese Stützkräfte großflächig auf das Becken übertragen. Durch den Schlitz im dünnwandigen Bereich kann sich der dickwandige Bereich sogar - unter Einwirkung der Stützkräfte - besonders gut um den Femurkopf anlegen, so daß die Stützkräfte von einer relativ großen Fläche abgefangen und großflächig auf das Becken übertragen werden.

0187881

Dadurch wird auch den natürlichen Verhältnissen gut Rechnung getragen. Es hat sich nämlich gezeigt, daß der Hohlraum zur Aufnahme des Femurkopfes bei einer natürlichen Acetabulumpfanne keineswegs in sich völlig starr ist, sondern bei entsprechenden Belastungen gewissen Verformungen unterliegt, die zumindest im sogenannten Mikrobereich liegen, sich aber dennoch in beachtlicher Weise auf die Lagerstelle Femurkopf/Acetabulumpfanne auswirken. Denn im völlig unbelasteten Zustand umschließt eine natürliche Acetabulumpfanne einen natürlichen Femurkopf nur scheinbar "satt", d. h. mehr oder weniger an sämtlichen Stellen flächig anliegend. Vielmehr sind im allgemeinen unter natürlichen Gegebenheiten nur einige Berührungspunkte zwischen der Außenseite des Femurkopfes und der die Lagerschale für diesen bildenden Innenseite der Acetabulumpfanne vorhanden – allenfalls eine abschnittweise linienförmige Berührung –, und es kommt erst mit zunehmender Beckenbelastung zu einer gleichsam zangenförmigen Verformung einer natürlichen Acetabulumpfanne derart, daß sich diese flächig bzw. an größeren Flächenabschnitten an den Femurkopf anlegt, wobei dann ersichtlich gleichzeitg durch Vergrößerung der gegenseitigen

Kontaktfläche trotz entsprechender Steigerung der Belastung gegenüber schwacher Belastung oder sogar gegenüber dem Ruhezustand eine entsprechende Senkung der Flächenpressung aufgrund der Kontaktflächenvergrößerung erfolgt.

Dieses alles ist unter natürlichen Gegebenheiten offenbar insbesondere auch deswegen möglich, weil das natürliche Acetabulumgewebe keineswegs annähernd halbkugelförmig ausgebildet ist, sondern weil von dieser Halbkugel ein verhältnismäßig großer Abschnitt überhaupt nicht vorhanden ist, nämlich an der weiter oben bereits erwähnten Ausnehmung, die zugleich einen Pfad für den Nervus femuralis und den ihn begleitenden Sehnenstrang schafft.

Da die bekannten Pfannen es aufgrund ihrer konstruktiven Gegebenheiten nicht zulassen, daß sie die vorstehend beschriebene Bewegung bzw. Verformung des Acetabulumgewebes bei entsprechenden Beanspruchungen mitmachen, kann es ersichtlich trotz einer anfänglich guten Verbindung zwischen der Außenseite einer Prothesenpfanne und der nachgewachsenen Spongiosa bei entsprechenden Belastungen und hieraus resultierenden Verformungen des Beckens wieder zu Ablöseerscheinungen kommen, die unter ungünstigen Umständen zu einer vollständigen Lockerung der Prothesenschale führen können.

Aufgrund der wenigstens etwa bis zur Symmetrieachse, vorzugsweise bis über diese hinaus, geschlitzten erfindungsgemäßen Prothesenpfanne erhält diese auch im implantierten Zustand völlig andere Verformungsmöglichkeiten als dieses bei bisher bekannten Pfannen der Fall war, und zwar Verformungsmöglichkeiten, welche die Eigenschaften einer natürlichen Acetabulumpfanne weitgehend simulieren, so daß es auch bei stärkeren Belastungen, die zu entsprechend starken elastischen Beckenverformungen führen, nicht zu den vorstehend beschriebenen Ablöseerscheinungen zwischen der Außenseite der Pfanne und dem Beckengewebe kommen kann, wobei durch entsprechende Dimensionierung der Schlitzbreite darüber hinaus sogleich sichergestellt werden kann, daß auch die bezüglich des Nervus femoralis beschriebenen Schwierigkeiten vermieden sind.

Die Schlitzbreite beträgt bevorzugt etwa 1 bis 3 cm, wobei es sich als zweckmäßig herausgestellt hat, wenn der Schlitz an seinem dem Pfannenrand abgekehrten Ende abgerundet ist, und zwar vorzugsweise mit einem Radius, der im wesentlichen etwa seiner halben Breite entspricht, wie dieses weiter unten anhand eines Ausführungsbeispiels noch weiter erläutert und dargestellt ist.

In besonders bevorzugter Ausgestaltung der vorliegenden Erfindung ist vorgesehen, daß der im wesentlichen halbkugelförmige Hohlraum in an sich bekannter Weise exzentrisch zu der von der Außenseite der Pfanne im wesentlichen gebildeten Halbkugel angeordnet ist, wobei der Schlitz im wesentlichen symmetrisch zu der dünnsten Stelle des Pfannenrandes angeordnet ist. Hierzu wird darauf verwiesen, daß eine exzentrische Anordnung des Hohlraums zur Außenseite aus der weiter oben angeführten Druckschrift an sich bekannt ist, daß bei dieser bekannten Pfanne aber die erwähnte Ausnehmung am Pfannenrand relativ zu der dünnsten bzw. dicksten Stelle der Pfanne anders angeordnet ist, da sich bei der bekannten Pfanne der Rand des schräg angeschnittenen Teilabschnittes von der Zone der größten Wandstärke bis
zur Zone der geringsten Wandstärke erstrecken soll.

Erfindungsgemäß beträgt die größte Wandstärke der
Pfanne etwa 4 bis 8, vorzugsweise ca. 6 mm, wobei die
Exzentrizität zwischen Hohlraum und Außenseite etwa
gleich der halben größten Wandstärke sein kann,
so daß die theoretische  dünnste Wandstärke - ohne
Berücksichtigung des symmetrisch von dieser Stelle
zur Symmetrieachse verlaufenden Schlitzes - praktisch
gerade Null ist, obwohl es selbstverständlich auch
möglich ist, diese Wandstärke, die bei der fertigen
Prothese aufgrund des Schlitzes ja nicht mehr vorhanden ist und hier mithin lediglich eine geometrische
Diskussionsgröße darstellt, beispielsweise 1 oder 2 mm
stark auszuführen.

Die Außenseite der Pfanne ist bevorzugt in an sich
bekannter Weise mit einer reliefartigen Profilierung
versehen, wie sie weiter unten noch beschrieben ist,
wobei es sich als zweckmäßig herausgestellt hat, wenn
darüber hinaus an der Außenseite noch wenigstens ein
Verankerungszapfen vorhanden ist, mit dem die Pfanne
- vorzugsweise völlig zementfrei - in dem entsprechend
vorbereiteten, d.h. ausgefrästen Acetabulumgewebe zu
verankern ist, wobei auch bevorzugte Ausgestaltungen
dieses Verankerungszapfens weiter unten noch im
einzelnen erläutert sind.

Außer dem Verankerungszapfen (und der bereits erwähnten
reliefartigen Strukturierung der Außenseite) sind bevorzugt noch weitere Befestigungsstellen vorgesehen,
die jeweils aus einem flügelartigen Vorsprung bestehen, wie dieses weiter unten auch noch im einzelnen
erläutert ist.

Weitere bevorzugte Ausgestaltungen der vorliegenden Erfindung sind in den Unteransprüchen beschrieben.

Die Erfindung ist nachstehend an Ausführungsbeispielen
unter Bezugnahme auf eine Zeichnung weiter erläutert.
Es zeigt:

Fig. 1     eine Draufsicht (von unten) auf eine erfindungsgemäße Pfannenprothese (etwa im
Maßstab (2:1) in Richtung des Pfeiles I
in Fig. 2 gesehen;

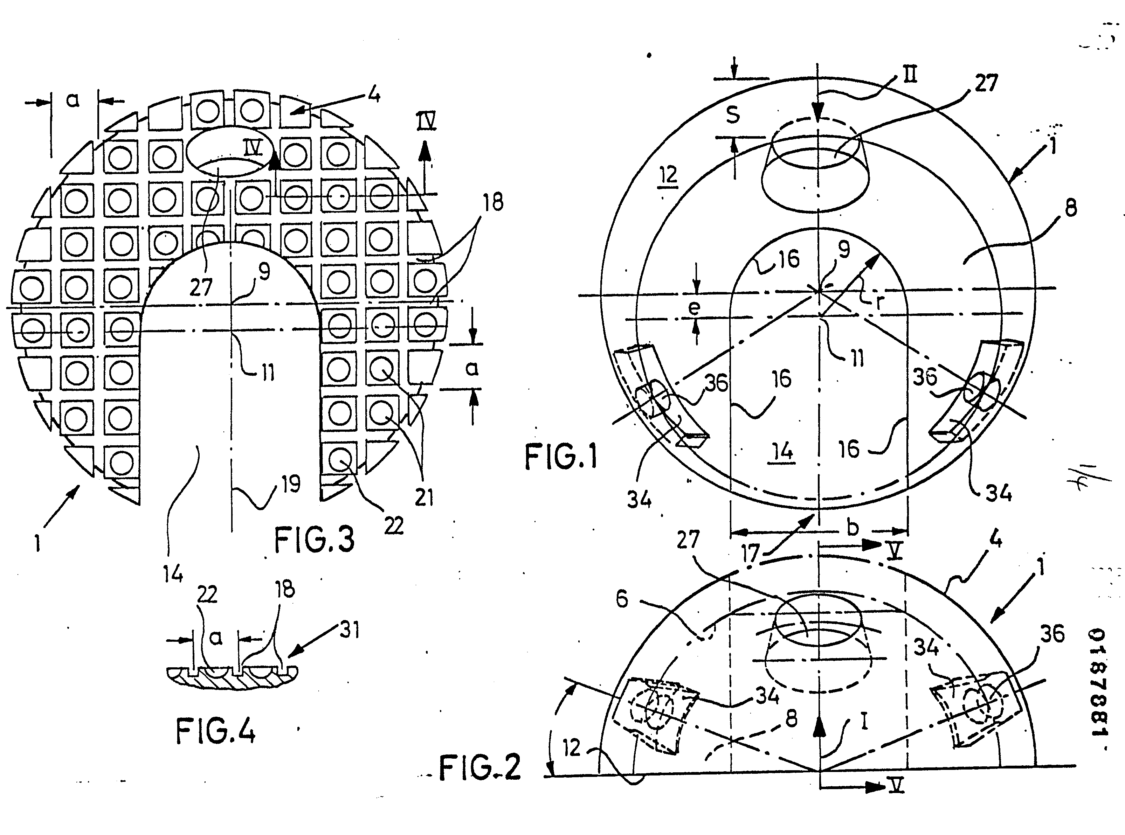

Fig. 2     eine seitliche Draufsicht auf die Pfanne
gemäß Fig. 1 in Richtung des Pfeiles II
in Fig. 1 gesehen;

Fig. 3     eine Draufsicht (von oben bzw. außen)
auf die Pfanne gemäß den Fig. 1 und 2;

Fig. 4     einen Teilausschnitt der rasterartigen
Profilierung der in den Fig. 3 erkennbaren Außenseite in Richtung der Schnittlinie IV-IV in Fig. 3 gesehen;

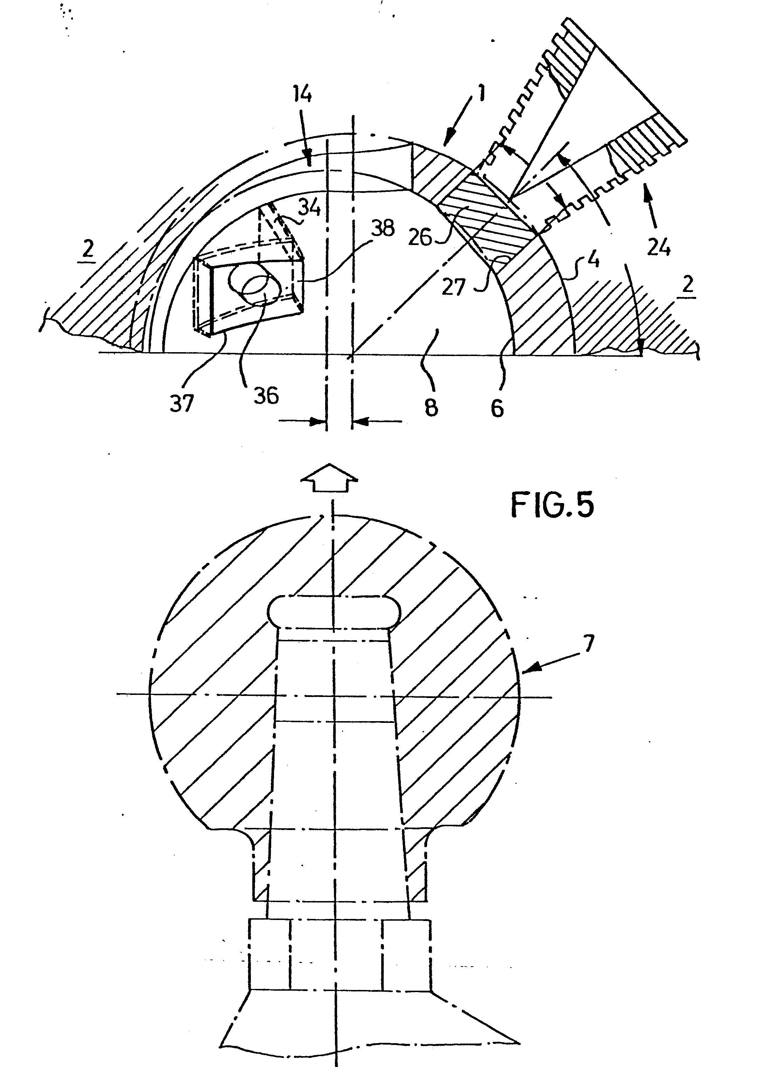

Fig. 5     eine Schnittansicht der Pfanne gemäß
den Fig. 1 bis 4 in Richtung der Schnittlinie V-V gesehen mit angebrachtem Verankerungszapfen und flügelartigem Verankerungsvorsprung, wobei in einer teilweisen Explosionsdarstellung mit strichpunktierten Linien zugleich ein kugelförmiger Femurkopf sowie ein Teil des
Femurschaftes dargestellt sind;

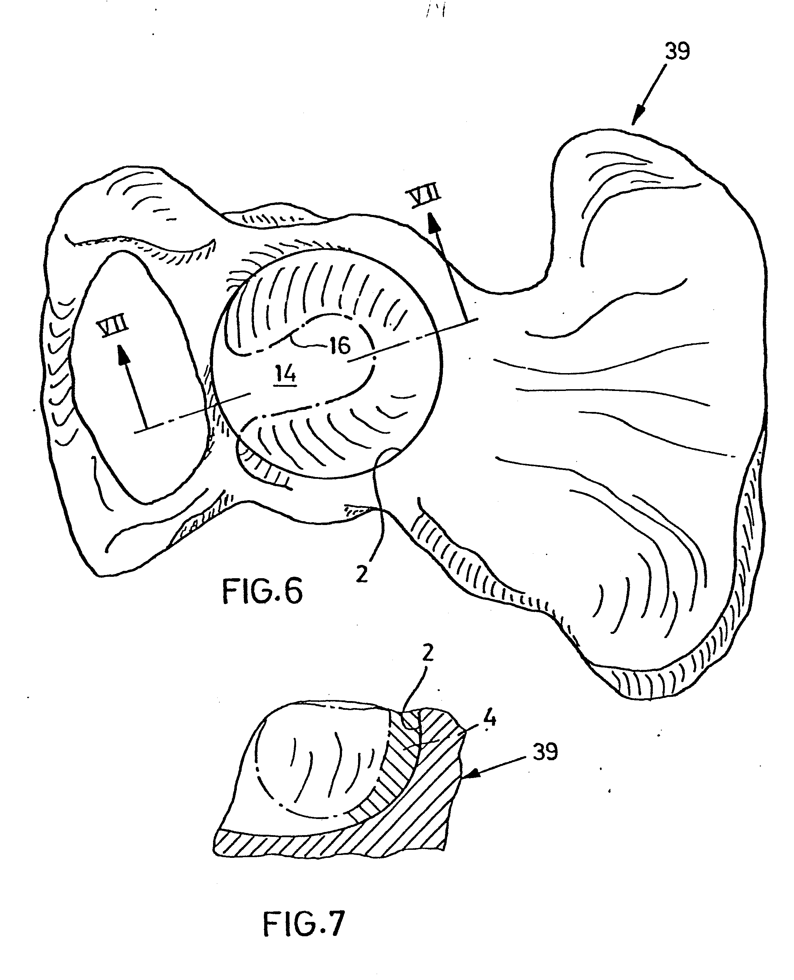

Fig. 6    eine etwas vereinfachte Draufsicht auf
          einen Beckenknochen;

Fig. 7    einen Teilschnitt durch das Becken gemäß
          Fig. 6 in Richtung der Schnittlinie
          VII-VII gesehen;

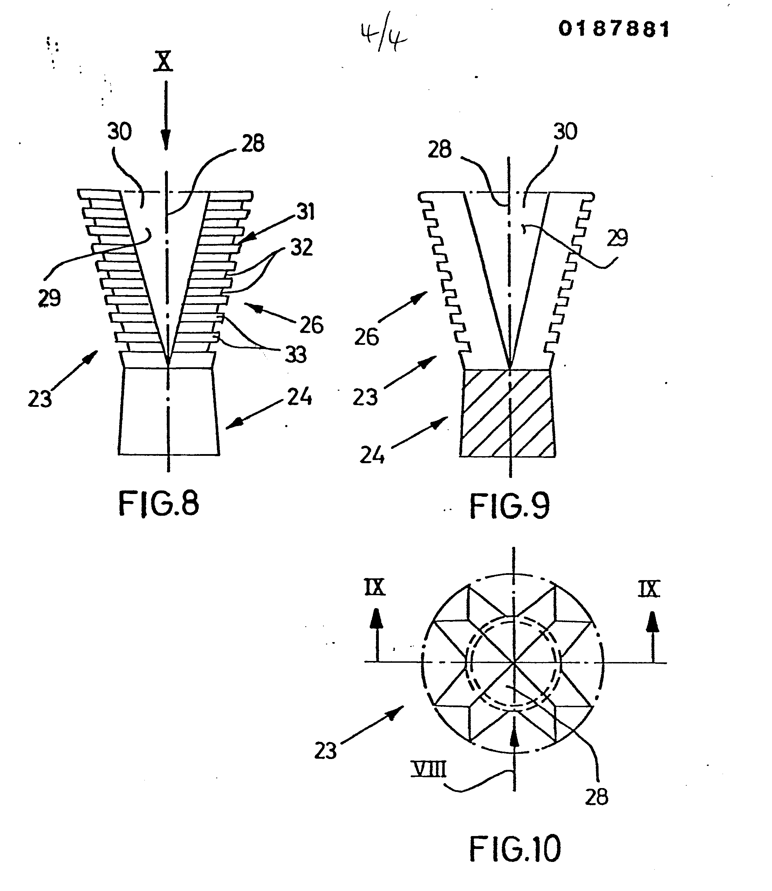

Fig. 8    eine Seitenansicht des Verankerungszapfens in Richtung des Pfeiles VIII in
          Fig. 10 gesehen;

Fig. 9    einen Schnitt durch den Verankerungszapfen
          gemäß Fig. 10 in Richtung der Schnittlinie
          IX-IX gesehen; und

Fig. 10   eine Draufsicht auf den Verankerungszapfen
          gemäß den Fig. 8 und 9 in Richtung des
          Pfeiles X in Fig. 8 gesehen.

Die Fig. 1 bis 5 der Zeichnung zeigen eine im ganzen mit
1 bezeichnete Acetabulumpfanne für eine Hüftgelenk-
Endoprothese, die zementfrei in dem in Fig. 5 angedeuteten mit 2 bezeichnetem Acetabulumgewebe eines Beckenknochens zu verankern ist. Die Pfanne 1 besteht im wesentlichen aus einer an ihrer Außenseite 3 im wesentlichen halbkugelförmigen Kunststoffschale 4, deren von
ihrer Innenseite 6 begrenzter, als Aufnahme- und damit
als Lagerschale für einen kugelförmigen Femurkopf 7
(s. Fig. 5) dienender Hohlraum 8 ebenfalls halbkugelförmig ausgebildet ist; allerdings liegen die Halbkugelflächen der Außenseite 3 bzw. der Innenseite 6
der Kunststoffschale 4 exzentrisch zueinander, und
zwar ist die Symmetrieachse 9 der halbkugelförmigen
Außenseite 3 um das Maß e = 2 mm zu der Symmetrieachse
11 der Innenseite 6 bzw. des Hohlraums 8 versetzt angeordnet, wobei die größte Wandstärke s am Rand 12
der Schale 4 6mm beträgt und stetig zur dünnsten Stelle
hin abnimmt.

An dem in der Meridianebene 13 der Außenseite 3 bzw.
des Hohlraums 8 verlaufenden Rand 12 der Schale 4
bzw. der Pfanne 1 ist eine nachstehend auch als
Schlitz bezeichnete schlitzförmige Ausnehmung 14 vorhanden. Der Schlitz 14 erstreckt sich, wie insbesondere
aus den Fig. 1 und 3 erkennbar ist, in der Draufsicht
auf die Pfanne 1 in Richtung der Symmetrieachse 9
bzw. 11 nicht nur über die Symmetrieachse 11, sondern
sogar auch noch über die Symmetrieachse 9 hinaus und
ist an seinem dem Rand 12 abgekehrten Ende mit einem
Radius r abgerundet, der gleich der halben Breite b
des Schlitzes 14 ist.

Die der Innenseite 6 der Pfanne 1 zugekehrten Kanten
16 des Schlitzes 14 sind mit einem kleinen Radius abgerundet bzw. angefast, um die Laufeigenschaften des
Femurkopfes 7 in der Pfanne 1 zu optimieren.

Die Breite b des Schlitzes 14 beträgt 20 mm. Der
Schlitz 14 ist symmetrisch zu der dünnsten Stelle 17
des Pfannenrandes 12 bzw. der Schale 4 angeordnet,
so daß die dickste Stelle mit einer Wandstärke s von
6 mm logischerweise nicht nur der dünnsten Stelle 17,
sondern auch dem abgerundeten Ende des Schlitzes 14 gegenüberliegt.

Wie die Fig. 3 und 4 erkennen lassen, ist die Außenseite 3 der Kunststoffschale 4 mit einer reliefartigen bzw. rasterförmigen Profilierung versehen, die durch parallel sowie in senkrecht zueinander verlaufende Nuten 18 ausgebildet ist, die mit gegenseitigem Abstand a zueinander angeordnet sind (s. Fig. 4). Die Nuten 18 verlaufen z.T. parallel und z.T. senkrecht zu der Mittelachse 19 des Schlitzes 14 bzw. der Schale 4. Die zwischen den Nuten 18 befindlichen Noppen 21 sind in der Draufsicht überwiegend quadratisch und weisen überwiegend jeweils eine nach außen hin offene Ausnehmung 22 auf, wobei die Ausnehmungen 22 halbkugelförmig ausgebildet sind.

Wie aus Fig. 5 erkennbar ist, steht von der Außenseite 3 der Schale 4 im dickwandigen Bereich der Pfanne 1 ein Verankerungszapfen 23 vor, der in verschiedenen Ansichten auch in den Fig. 8 bis 10 dargestellt ist. Der Verankerungszapfen 23 besitzt einen Befestigungsabschnitt 24 und einen Verankerungsabschnitt 26. Der Befestigungsabschnitt 24 ist in eine Bohrung 27 der Schale 4 eingesetzt und fest mit dieser verbunden. Hierfür ist der Verankerungsabschnitt 26 zu seinem freien Ende hin leicht konisch (siehe Fig. 8). Zwecks Verbindung des Befestigungsabschnittes 24 in der Bohrung 27 wird die Schale im Heißwasserbad erwärmt und der Befestigungsabschnitt 24 des Verankerungszapfens 23 wird im erwärmten Zustand der Bohrung 27 so eingeführt, daß er nicht über die Innenseite 6 der Schale 4 vorsteht. Nach dem Erkalten ergibt sich eine außerordentlich feste Verbindung zwischen Verankerungszapfen 23 und Schale 4, die praktisch die Eigenschaften einer entsprechend integral ausgebildeten Pfanne 1 hat.

Der vorstehende Verankerungsabschnitt 26 des Verankerungszapfens 23 ist ebenfalls kegelförmig ausgebildet, wobei die Konizität allerdings umgekehrt ist als am Verankerungsabschnitt 26. Er besitzt einen symmetrisch zu seiner Längsachse 28 verlaufenden kreuzförmigen Schlitz 29 und ist an seinem freien Ende mit einer etwa kegelförmigen Ausnehmung 30 versehen. Darüber hinaus weist der Verankerungsabschnitt 26 an seiner Außenseite eine Profilierung 31 auf, die aus zueinander parallelen Nuten 32 und dazwischen liegenden Vorsprüngen 33 besteht. Eine entsprechende Profilierung kann auch an der Innenseite der vier den Verankerungsabschnitt 26 bildenden Stränge 26' vorgesehen sein.

Wie insbesondere Fig. 5 erkennen läßt, ist an der Außenseite 3 der Kunststoffschale 4 und damit der Pfanne 1 jeweils beidseitig und spiegelsymmetrisch zu der Bohrung 27 ein nachstehend auch kurz als Flügel bezeichneter flügelartiger Vorsprung 34 vorhanden, wobei die beiden Flügel 34, die in den Fig. 1 und 2 noch in nicht aus der sphärischen Kugelebene herausgeklappte Flügel dargestellt sind, zusammen mit der Bohrung 27 bzw. dem Verankerunszapfen 23 drei Verankerungspunkte bilden, die – zusätzlich zu der Profilierung 31 an der Außenseite 3 – Pfanne 1 in dem Acetabulumgewebe 2 formschlüssig zu verankern, wobei diese drei Befestigungspunkte 34, 27, 34 in der Draufsicht gemäß Fig. 1 etwa an den Eckpunkten eines gleichseitigen Dreiecks liegen. Die Flügel 34 besitzen jeweils eine Durchgangsbohrung 36, in welche im implantierten Zustand Spongiosa einwachsen kann. Sie sind jeweils durch einen etwa U-förmigen Einschnitt in die Wandung der Schale 4 gebildet, der in Fig. 5 mit einer dicken schwarzen Linie angedeutet und mit 37 bezeichnet ist, und um ihre Verbindungsstelle 38 zur Pfannen- bzw. Schalenwandung nach entsprechender Wärmebeaufschlagung nach außen geschwenkt, wie dieses in Fig. 5 dargestellt ist.

Das Einpflanzen in den Beckenknochen 39 (s. Fig. 6,7), genauer gesagt das entsprechend mit einem Kugelfräser ausgefräste Acetabulum, welches in der Handskizze gemäß Fig. 6 und 7 in einem noch nicht ausgefrästen Zustand gezeigt ist, erfolgt, wie dieses in Fig. 6 in einer strichpunktierten Linie angedeutet ist, welche etwa den Rand bzw. die Kanten 16 des Schlitzes 14 kennzeichnen sollen.

Zunächst einmal ist anhand der Darstellung gemäß den Fig. 6 und 7 zu erkennen, daß das natürliche Acetabulum 2 keineswegs etwa geometrisch die Form einer Halbkugel aufweist, sondern lediglich eines Abschnittes einer Halbkugel, der größenordnungsmäßig nur etwas mehr als die Hälfte einer Halbkugel beträgt, während die andere Hälfte der Halbkugel im natürlichen Zustand überhaupt nicht vorhanden ist. Diesem Umstand trägt die erfindungsgemäße Pfanne 1 in optimaler Weise durch den Schlitz 14 Rechnung, der dazu führt, daß auch die Prothesenpfanne 1 im implantierten Zustand völlig innerhalb der Geometrie der natürlichen Acetablumpfanne verschwindet und nicht über diese nach außen vorsteht, so daß der Nervus femoralis und der mit diesem verlaufende Sehnenstrang ungehindert an dieser Stelle vorbei geleitet werden können, wie dieses auch im natürlichen Zustand der Fall ist.

Darüber hinaus ergeben sich nach der Implantation ersichtlich ganz ähnliche Verformungsverhältnisse als dieses bei der natürlichen Acetabulumpfanne der Fall ist. Dieses beginnt einmal schon damit, daß die vorgesehene Wandstärke von 6 mm etwa der üblicherweise entfernten maximalen Gewebestärke entspricht. Weiterhin beruht dieses darauf, daß sich die Pfanne 1 im implantierten Zustand zusammen mit dem Beckenknochen 39 verformen kann, ohne daß es zu Relativbewegungen zwischen beiden kommt.

Die Implantation erfolgt im wesentlichen derart, daß zunächst einmal für den Verankerungszapfen 23 nach der Ausfräsung des Acetabulumgewebes 2 eine entsprechende Bohrung in dieses eingebracht wird, und daß ggf. Aussparungen für die Flügel 34 präpariert werden. Das Einführen des Verankerungsabschnittes 26 des Verankerungszapfens 23 erfolgt derart, daß der Operateur die vier Stränge des Verankerungsabschnittes 26 mit einer Ringzange umfaßt und diese in Richtung auf die Längsachse 28 zusammendrückt, wobei in diesem Zustand in einer Nut 32 ein geschlossener Faden angebracht wird bzw. vorher angebracht worden ist, der sich später auflöst. Nach dem Auflösen federn die vier Stränge des Befestigungsfeldes 26 dann in der aus den Fig. 5 und 8 bis 10 erkennbaren Weise auseinander und sorgen aufgrund des Formschlusses - unterstützt durch die Profilierung 31 - alsbald für eine feste Verankerung, und zwar im Zusammenwirken mit den Flügeln 34, die im implantierten Zustand nach außen vorstehen und sich in das Acetabulumgewebe 2 hineinerstrecken. Im Verlaufe der Heilzeit wächst sodann auch noch Spongiosa in die Profilierung 31 hinein, so daß sich insgesamt eine außergewöhnlich gute mechanische Verbindung zwischen dem Acetabulumgewebe 2 und der Außenseite 3 der Pfanne 1 ergibt.

Wird der Beckenknochen 39 und damit das Acetabulumgewebe 2 später Beanspruchungen ausgesetzt, die zu einer Verformung auch des Acetabulums führen, so macht die Pfanne 1 diese Verformungen aufgrund ihrer Elastizitätseigenschaften ohne weiteres mit, d.h. es erfolgt praktisch das Gleiche, was im natürlichen Zustand erfolgt: Wird nämlich das Becken entsprechend belastet, so kommt es zu einer flächigeren Anlage der Innenseite 6 der Schale 4 an dem Femurkopf 7 als im unbelasteten Zustand, wobei auch extreme Torsionsbeanspruchungen des Beckens zumindest dann entsprechend Berücksichtigung finden, wenn die Pfanne 1 unter einer gewissen Vorspannung implantiert wird.

Gleichzeitig ergeben sich optimale Lauf- und Abstützeigenschaften, da an derjenigen Stelle, an welcher die größten Druckkräfte aufzunehmen sind, auch die größten Materialstärken vorhanden sind, während dort, wo praktisch keine oder allenfalls geringe Lagerkräfte aufzunehmen sind, die Wandstärke entsprechend geringer (bzw. schließlich Null) ist, so daß insgesamt kein überflüssiges Material vorhanden ist und die Elastizitätseigenschaften der erfindungsgemäßen Acetabulumpfanne    insgesamt den natürlichen Gegebenheiten weitgehend entsprechen.

# EISENFÜHR & SPEISER
Patentanwälte · European Patent Attorneys

**0187881**

Unser Zeichen: O 78

Anmelder/Inh.: orthoplant

Aktenzeichen: Neuanmeldung

Datum: 15. Januar 1985

Patentanwälte
Dipl.-Ing. Günther Eisenführ
Dipl.-Ing. Dieter K. Speiser
Dr.-Ing. Werner W. Rabus
Dipl.-Ing. Detlef Ninnemann
Dipl.-Ing. Jürgen Brügge

orthoplant Endoprothetik GmbH, Leerkämpe 12, 2800 Bremen 1

------------------------------------------------------------

Hüftpfanne

------------------------------------------------------------

1. Hüftpfanne für eine Hüftgelenk-Endoprothese zur zementfreien Verankerung im menschlichen Acetabulumgewebe, bestehend aus einer Schale aus Kunststoff, die eine halbkugelförmige Außenfläche und eine einen halbkugelförmigen Hohlraum bildende Innenfläche besitzt, die exzentrisch zur Außenfläche angeordnet ist und dadurch einen dünnwandigen und einen dickwandigen Pfannenbereich bildet, und wobei an dem Rand der Pfanne eine Ausnehmung vorhanden ist, dadurch gekennzeichnet, daß die Ausnehmung im dünnwandigen Bereich der Pfanne in der Art eines Schlitzes (14) ausgebildet ist, der sich wenigstens bis zur Symmetrieachse (11) der Innenfläche (6) hin erstreckt.

WWR/kl/dg

Martinistraße 24    Telefon         Telecopierer      Telex              Datex-P
D-2800 Bremen 1    0421-32 80 37    0421-32 68 34    2 44 020 fepat d    44 421040 311

2. Pfanne nach Anspruch 1, dadurch gekennzeichnet, daß sich der Schlitz (14) bis über die Symmetrieachse (11) der Außenfläche (3) hinaus erstreckt.

3. Pfanne nach Anspruch 1 oder 2, dadurch gekennzeichnet, daß der Schlitz (14) an seinem dem Pfannenrand (12) abgekehrten Ende abgerundet ist.

4. Pfanne nach einem der vorhergehenden Ansprüche, dadurch gekennzeichnet, daß die Breite (b) des Schlitzes (14) etwa 1 bis 3 cm beträgt.

5. Pfanne nach einem der vorhergehenden Ansprüche, dadurch gekennzeichnet, daß der Schlitz (14) im wesentlichen symmetrisch zu der dünnsten Stelle (17) des Pfannenrandes (12) angeordnet ist.

6. Pfanne nach Anspruch 4 oder 5, dadurch gekennzeichnet, daß die Exzentrizität (e) zwischen der Innenfläche (6) und der Außenfläche (3) der Pfanne (1) etwa gleich der halben größten Wandstärke (s) der Pfanne ist.

7. Pfanne nach einem oder mehreren der vorhergehenden Ansprüche, dadurch gekennzeichnet, daß die Außenfläche (3) mit einer reliefartigen bzw. rasterförmigen strukturierten Profilierung (18, 21) versehen ist.

8. Pfanne nach einem der vorhergehenden Ansprüche, dadurch gekennzeichnet, daß von der Außenfläche (3) wenigstens ein Verankerungszapfen (23) vorsteht.

9. Pfanne nach Anspruch 8,
dadurch gekennzeichnet, daß der Verankerungszapfen (23) mit einem Befestigungsabschnitt (24) in eine Bohrung (27) der Schale (6) eingesetzt und fest mit dieser verbunden ist.

10. Pfanne nach Anspruch 12 oder 13,
dadurch gekennzeichnet, daß der vorstehende Verankerungsabschnitt (26) des Verankerungszapfens (23) kegelförmig ausgebildet ist und sich von seinem mit dem Befestigungsabschnitt (24) fest verbundenen Ende zu seinem freien Ende hin konisch erweitert.

11. Pfanne nach einem der Ansprüche 12 bis 14,
dadurch gekennzeichnet, daß der Verankerungsabschnitt (26) des Verankerungszapfens (23) einen symmetrisch zu seiner Längsachse (28) angeordneten kreuzförmigen Schlitz (29) aufweist, der an seinem freien Endabschnitt eine zentrische Ausnehmung (30) aufweist; und daß die einzelnen Stränge des Verankerungsabschnittes (26) an ihrer Innenseite und Außenseite mit einer Profilierung versehen sind.

12. Pfanne nach einem der Ansprüche 8 bis 11,
dadurch gekennzeichnet, daß mindestens ein Verankerungszapfen (23) im dickwandigen Bereich der Pfanne vorgesehen ist.

13. Pfanne nach einem der vorhergehenden Ansprüche,
dadurch gekennzeichnet, daß zwei flügelartige Ansätze (34) im wesentlichen symmetrisch und mit Abstand zur Mittelachse (19) des Schlitzes (14) angeordnet sind.

1/4    0187881

FIG.1  FIG.2  FIG.3  FIG.4

FIG.5

0187881

FIG.6

FIG.7

0187881

FIG.8

FIG.9

FIG.10

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | DE-A-1 806 323   (WALDEMAR) <br> * Abbildungen; Seite 5, Zeile 5 - Seite 6, Zeile 13 * | 1-3 | A 61 F   .2/34 |
| Y | EP-A-0 051 729   (WALDEMAR) <br> * Abbildungen; Seite 4, Zeile 24 - Seite 5, Zeile 9 * | 1-3 | |
| A | EP-A-0 028 546   (TEINTURIER) | | |
| A | EP-A-0 083 708   (SULZER) | | |
| A | DE-A-2 205 400   (SULZER) | | |
| A | DE-A-2 250 501   (FISCHER) | | RECHERCHIERTE SACHGEBIETE (Int. Cl.4) <br><br> A 61 F |
| E | DE-A-3 331 191   (ORTHOPLANT) <br> * Insgesamt * | 1-13 | |

Der vorliegende Recherchenbericht wurde für alle Patentansprüche erstellt.

| Recherchenort <br> DEN HAAG | Abschlußdatum der Recherche <br> 06-09-1985 | Prüfer <br> STEENBAKKER  J. |
|---|---|---|